(19) Europäisches Patentamt
European Patent Office
Office européen des brevets

(11) Veröffentlichungsnummer: **0 279 425 B1**

# (12) EUROPÄISCHE PATENTSCHRIFT

(45) Veröffentlichungstag der Patentschrift: **04.08.93**

(51) Int. Cl.[5]: **A61K 31/55**

(21) Anmeldenummer: **88102294.1**

(22) Anmeldetag: **17.02.88**

## (54) Verwendung von Benzothiazepin-Derivaten, insbesondere Diltiazem zur Herstellung von Arzneimitteln zur Protektion von Lymphozyten.

(30) Priorität: **18.02.87 DE 3705117**

(43) Veröffentlichungstag der Anmeldung:
**24.08.88 Patentblatt 88/34**

(45) Bekanntmachung des Hinweises auf die Patenterteilung:
**04.08.93 Patentblatt 93/31**

(84) Benannte Vertragsstaaten:
**AT BE CH DE FR GB IT LI LU NL SE**

(56) Entgegenhaltungen:
**EP-A- 0 116 967**
**GB-A- 2 120 941**
**US-A- 4 663 317**

**CANCER RESEARCH, Band 45, Nr. 3, März 1985, Seiten 1005-1007; S.-I.AKLYAMA et al.: "Potentiation of cytotoxic activity immunotoxins on cultured human cells"**

**DTSCH. MED. WSCHR., Band 111, Nr. 36, 1986, Seiten 1363-1367, Georg Thieme Verlag Stuttgart, New York, US; K. WAGNER et al.: "Protektiver Einfluss des Calciumantagonisten Diltiazem auf das akute Nierenversagen nach Nierentransplantation"**

**THE JOURNAL OF IMMUNOLOGY, Band 133, nr. 6, Dezember 1984, Seiten 2904-2909, The American Association of Immunologists, US; D.L. BIRX et al.: "The interference of T cell activation by calcium channel blocking agents"**

(73) Patentinhaber: **GÖDECKE AKTIENGESELLSCHAFT**
**Salzufer 16**
**W-1000 Berlin 10(DE)**

(72) Erfinder: **Weiershausen, Ute**
**Bundesstrasse 70**
**W-7803 Gundelfingen(DE)**
Erfinder: **Ganser, Volker, Dr.**
**Sulzburgerstrasse 22**
**W-7800 Freiburg(DE)**
Erfinder: **Satzinger, Gerhard, Dr.**
**Im Mattenbühl 7**
**W-7809 Denzlingen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

AIDS (Acquired Immune Deficiency Syndrome) ist eine sehr ernst zu nehmende Infektionskrankheit, gegen die bisher wirksame Mittel nicht aufgefunden werden konnten.

Aus der DE-PS 18 05 714 sind Benzothiazepine bekannt geworden, deren Tranquilizerwirksamkeit dem Chlordiazepoxid überlegen sein sollte. Später fand man, daß den dort beschriebenen Verbindungen mindestens teilweise eine calciumantagonistische Wirksamkeit zugeschrieben werden kann. Das 2-(4-Methoxyphenyl)-3-acetoxy-5(β-dimethylaminoethyl)-2,3-dihydro-1,5-benzothiazepin-4(5H)-on (Diltiazem), insbesondere dessen Hydrochlorid gehört mittlerweile zu den klassischen Calciumantagonisten und wird weltweit eingesetzt. Es ist auch auf GB-A-2120941 bekannt, daß Diltiazem die Wirkung von Zytostatika erhöhen kann.

Es wurde nun gefunden, daß der Verbindungsklasse der DE-PS 18 05 714 noch eine weitere hochinteressante Indikation zugeschrieben werden kann, nämlich eine überraschende Schutzwirkung gegenüber immunkompetenten Lymphozyten.

Gegenstand der vorliegenden Patentanmeldung ist daher die Verwendung von Benzothiazepin-Derivaten der allgemeinen Formel I

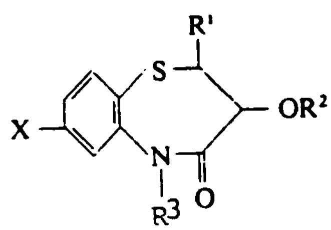

I

in der $R^1$ Wasserstoff oder einen Phenylrest, der gegebenenfalls durch eine Methylgruppe, 1 bis 3 Methoxygruppen oder 1 bis 2 Chloratome substituiert ist, $R^2$ ein Wasserstoffatom oder die Acetylgruppe, X ein Wasserstoffatom oder ein Chloratom und $R^3$ ein Wasserstoffatom oder die Gruppe

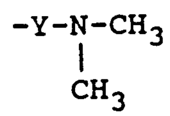

darstellt, wobei Y einen Alkylenrest mit 2 oder 3 Kohlenstoffatomen bedeutet
und deren pharmazeutisch verwendbaren Salzen zur Herstellung von Arzneimitteln zur Verhinderung der durch lymphozytotrope Noxen oder Arzneimittel, mit Ausnahme von Zytostatika, hervorgernfere toxische Wirkungen auf lymphozyten. Menschen und höherer Säugetiere.

Besonders bevorzugt ist die Verwendung von Diltiazem-Hydrochlorid.

Die Verbindungen der allgemeinen Formel I stellen demnach Mittel dar, mit deren Hilfe durch Arzneimittel oder lymphozytotrope Viren bedingte toxische Wirkungen auf Lymphozyten verhindert werden können. Sie ermöglichen ein neues Therapiekonzept im Sinne einer "selektiven Lymphozytenprotektion".

Unter lymphozytotoxischen Arzneimitteln werden im vorliegenden Zusammenhang Substanzen unterschiedlicher Wirkstoffklassen (z.B. Cytostatika, Antibiotika oder Antihypertensiva) verstanden, sofern diese entweder die Zahl der im Blut kreisenden Lymphozyten vermindern oder ihre immunologischen Funktionen beeinträchtigen. Beide Effekte führen zu einem mehr oder weniger ausgeprägten Versagen der Immunabwehr mit der Folge einer erhöhten Infektionsbereitschaft des Organismus. Bei dem erstgenannten Wirkungstyp, dem insbesondere lymphozytopenisch wirkende Cytostatika wie Cyclophosphamid zuzuordnen sind, kommt es aufgrund einer die Zellproliferation hemmenden Aktivität in den meisten Fällen zur Anhäufung unreifer und immuninkompetenter Lymphozyten-Vorstufen im Knochenmark, während gleichzeitig die Zahl der im Blut kreisenden reifen und immunologisch funktionsfähigen Lymphozyten abnimmt. Der zweite genannte Wirkungstyp führt bei unveränderter Lymphozytenzahl zu einer Veränderung von Lymphozytenfunktionen. Dieser Effekt kann auf sehr unterschiedliche Weise zustandekommen. Das Immunsuppressivum Cyclosporin A z.B. hemmt die Aktivierung der eigentlichen Abwehr-Lymphozyten (zytotoxische T-Lymphozyten) über eine Hemmung der Produktion des aktivierenden Botenstoffs durch die sogenannten T-Helferlymphozyten. Es wird bei dieser Substanz darüberhinaus auch eine Hemmung der Ausbildung jener

Rezeptoren auf zytotoxischen T-Zellen diskutiert, an welche der sie aktivierende Botenstoff bindet.

Auch durch reversible Blockierung dieser Rezeptoren durch nichtzytotoxische Substanzen erzielt man einen Schutz der Lymphozytenoberfläche gegenüber lymphozytotropen Noxen.

Unter lymphozytotropen Noxen versteht man im vorliegenden Fall auch lymphozytotrope Viren. Bekanntlich geht der eigentlichen Infektion der Zelle durch diese Erreger zunächst deren Bindung an bestimmte Rezeptoren auf der Zelloberfläche voraus, wonach der Rezeptor-Virus-Komplex dann in das Zellinnere eingestülpt wird. Lymphozytotrope Viren können ihre Targetzellen entweder immortalisieren, d.h. sie maligne transformieren oder deren Elimination durch virusinduzierte Autoimmunprozesse (HIV im Fall von T-Helferzellen) provozieren. Für die genannten lymphozytotropen Virusinfektionen gibt es bisher keine wirksame Medikation. Die Patienten erliegen im allgemeinen aufgrund der virusinduzierten massiven Immuninsuffizienz generalisierten Infekten.

Die besondere Problematik der lymphozytotropen Viren im Vergleich zu den nicht lymphozytotropen Viren besteht demnach darin, daß sie bevorzugt Zellen des Immunsystems infizieren und daher genau die Mechanismen ausschalten, welche für die Elimination dieser Viren selbst wie auch begleitender opportunistischer Erreger unverzichtbar sind.

Aufgabe der Erfindung ist es, nicht zytotoxische und darüberhinaus durch eine hohe allgemeine Verträglichkeit gekennzeichnete Substanzen zu finden, welche für Lymphozyten toxische Bindungspartner chemischer oder viraler Art von ihren spezifischen Rezeptoren auf Lymphozytenoberflächen verdrängen oder ihre Interaktion mit diesen Targetzellen über andere Mechanismen behindern.

Im Fall der lymphozytotoxischen Cytostatika kann damit die bei diesen Antitumormitteln dosislimitierende und die Patienten häufig vital gefährdende Immunsuppression verhindert werden.

Darüberhinaus kann man langfristig auch die bekannte Induktion von Sekundärtumoren durch Cytostatika beeinflussen, deren Entstehung durch die iatrogene Immunschwäche begünstigt wird.

Im Fall lymphozytotroper Viren können erfindungsgemäß die zur Anheftung an Lymphozytenoberflächen von den Erregern benutzten Rezeptoren auf untoxische Weise blockiert werden. Hierbei ist an eine limitierte Prophylaxe in Risikosituationen zu denken, aber auch bei bereits manifestem Infekt wird aufgrund der Verhinderung der Infektion weiterer, vom Knochenmark nachgelieferter Immunzellen langfristig eine Restitution der Immunkompetenz erwartet. Es ist dann z.B. die bei HIV-Infekten (AIDS-Infektion) bisher nicht zu erzielende Erhaltung des Lebens der Patienten möglich.

Bei Versuchen zur Antagonisierung der immunsuppressiven Wirkung der antineoplastisch wirkenden Substanz Dinalin [= 4-Amino-N-(2′-aminophenyl)-benzamid, vgl. EP-PS 0 116 967] in einem Hauttransplantationsmodell bei Ratten wurde erstmals nachgewiesen, daß Diltiazem in einer Dosierung von 200 mg/kg/Tag, intragastral verabreicht, die immunsuppressive Aktivität von Dinalin deutlich antagonisiert (Tab. 1, Abb. 1). Da die immunsuppressive Aktivität in diesem Modell über eine Inhibition zytotoxischer T-Lymphozyten bzw. deren Funktion zustandekommt, kann man auf einen Schutz dieser Zellpopulation unter Diltiazem gegenüber der antiproliferativen Wirkung dieses Antineoplastikums schließen. Daß Diltiazem einen Einfluß auf zytotoxische T-Lymphozyten haben muß, wurde auch dadurch deutlich, daß die Substanz, allein verabreicht, selbst eine mäßige Verzögerung der Transplantatabstoßung bewirkt (Tabelle 1).

Über die LDL-Rezeptoren werden die cholesterinreichen Lipidartikel des Serums (low density lipoproteins) in die Zellen eingeschleust. Das vermehrte Vorkommen dieser Rezeptoren auf Lymphozytenoberflächen ist möglicherweise in Verbindung mit deren hoher Teilungsrate zu sehen, die einen erhöhten Verbrauch des Membranbausteins Cholesterin mit sich bringt. Die bekannte Bindung von Diltiazem an die LDL Rezeptoren kann für seine Membranwirkung bzw. Verdrängungsmechanismen auch gegenüber viralen Bindungspartnern an Lymphozytenoberflächen verantwortlich sein. Die erhöhte LDL-Rezeptordichte auf Lymphozyten könnte auch die Selektivität der nachgewiesenen Schutzwirkung für die Lymphozytenpopulation erklären.

Die reversible nichtzytotoxische Arretierung der Lymphozytenproliferation und die Beeinflußung von Rezeptoren auf der Lymphozytenoberfläche können demnach Mechanismen der erfindungsgemäßen Lymphozytenprotektion gegenüber chemisch und viral bedingten lymphozytotropen Noxen durch die Verbindungen der allgemeinen Formel I darstellen.

Versuche zur Protektion von T-Lymphozyten gegenüber arzneimittelinduzierten lymphozytotoxischen Effekten wurden an einem Rattenhaut-Transplantationsmodell nach Waynforth (Experimental and Surgical Techniques in the Rat, Academic Press, 1980) modifiziert nach Heystek, durchgeführt. Es werden je 2 allogene Hauttransplantate histoinkompatibler Spender-Ratten auf Empfänger-Ratten übertragen. Die Transplantate werden mit einer Stanze gewonnen, präparativ vom Panniculus carnosus befreit und in ebenfalls ausgestanzte Transplantatbetten des Empfängers plaziert. Die Transplantate werden mit speziellem Verbandmaterial bedeckt und mit einer Bandage fixiert. Endpunkt der Untersuchung ist der Tag, an dem mindestens 50 % der Tiere beide Transplantate abgestoßen haben. Die immunsuppressive Aktivität eines

Wirkstoffs wird anhand der Verlängerung des normalen Transplantatüberlebens (Tage) der nicht behandelten Kontrolltiere bestimmt (Fig. 1, Tab. 1).

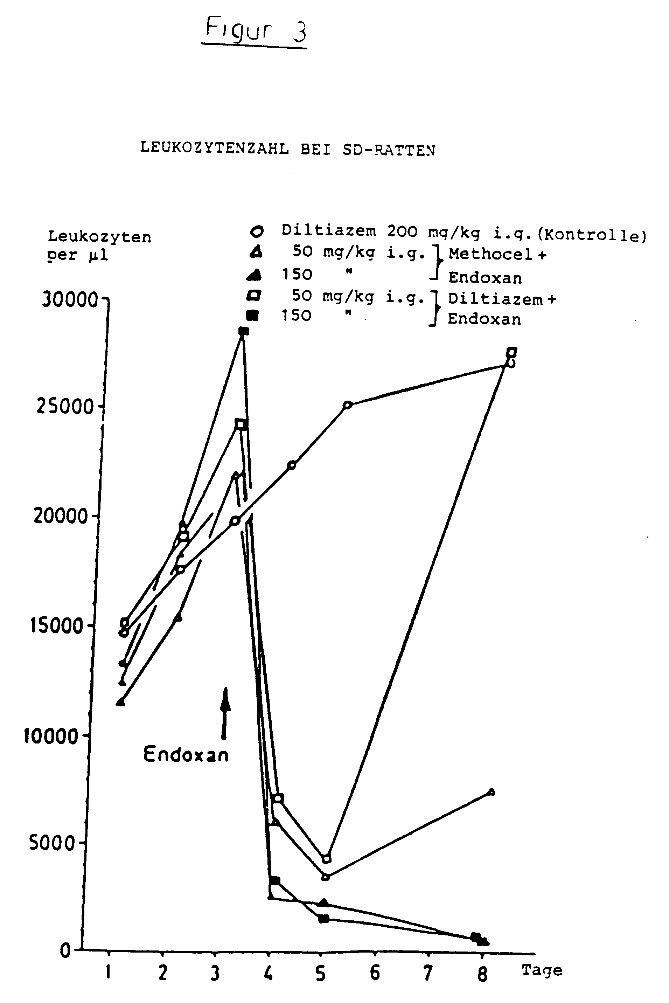

Weiterhin wurden Versuche zur Beeinflussung der Cyclophosphamid-induzierten Lymphopenie an Ratten durchgeführt. Eine Gruppe, die ausschließlich Diltiazem erhielt, diente als Kontrolle. Zwei Dosierungen von Cyclophosphamid, als Einzeldosen verabreicht, wurden mit einer Kombination des Cytostatikums mit Diltiazem verglichen, wobei der Kombinationsbehandlung eine 2-tägige Vorbehandlung mit Diltiazem vorausging. Die Bestimmung von Leukozyten- und Lymphozytenzahlen erfolgte im Blut, das jeweils am Morgen vor der Substanzgabe aus der Retroorbitalhöhle in Penthrane®-Kurznarkose entnommen wurde. Die Ergebnisse sind in den Figuren 2 und 3 dargestellt.

Der Nachweis der Lymphozytenprotektion gegenüber lymphozytotropen Viren wird in vitro anhand behandelter Humanlymphozyten oder Lymphozyten-Zellinien in Kultur und $^{125}$J-markiertem, hitzeinaktiviertem HIV erbracht. Nach Inkubation der Zellen mit dem Virus wird die Radioaktivität im Vergleich zu nicht behandelten Kontroll-Lymphozyten in einem Geigerzähler bestimmt. Als Positivstandard dient Diphenylhydantoin [Dtsch. Med. Wochensch. 25, S. 1001-1002 (1986)].

Am Beispiel des stark leukopenisch wirkenden Cytostatikums Cyclophosphamid konnte gezeigt werden, daß eine intragastrale Vorbehandlung mit Diltiazem in einer Dosierung von 200 mg/kg/Tag die durch hohe orale Einzeldosen von 15o mg/kg Cyclophosphamid bei Ratten induzierte massive Lymphozytopenie vollständig verhindert (Fig. 2). Die durch Cyclophosphamid ebenfalls induzierte Granulozytopenie wurde durch Diltiazem nicht beeinflußt (Fig. 3). Es handelt sich folglich um einen für Lymphozyten spezifischen Effekt.

Weiterhin wurde untersucht, ob Diltiazem die immunsuppressive Aktivität auch solcher Substanzen beeinflußt, bei welchen diese nicht über lymphopenische Wirkungen sondern über die Hemmung von Lymphozytenfunktionen zustandekommt. Cefradin hat keine lymphopenische aber eine starke immunsuppressive Wirkung. Eine gleichzeitige Behandlung der Tiere mit Diltiazem führte bei dieser Substanz in hoher Dosierung zu einer weitgehenden Aufhebung dieser Aktivität (Tabelle 1). Bei der Kombinationsbehandlung wurde Diltiazem jeweils vor der zu antagonisierenden Substanz, und zwar in mindestens halbstündigem Abstand, verabreicht.

Aufgrund der insgesamt durchgeführten Versuche ist es offensichtlich, daß die Verbindungen I, insbesondere Diltiazem in geeigneter Dosierung einen selektiven Schutz peripherer Lymphozyten, insbesondere T-Lymphozyten, gegenüber arzneimittelbedingten quantitativen und qualitativen Schädigungen dieser Zellpopulation ermöglichen. Insbesondere aufgrund der Antagonisierung der immunsuppressiven Wirkung von Cefradin, welche wohl auf Rezeptorebene abläuft, schließt dies den Schutz auch gegenüber viralen lymphozytotropen Bindungspartnern (z.B. HIV) in das Wirkungsspektrum der erfindungsgemäßen Substanzen ein.

Bei orientierenden Versuchen mit den Verbindungen der Formel I hat sich nämlich herausgestellt, daß diese über eine direkte Membranwirkung die Bindungsstellen der Zelle für das AIDS-Virus verändert, so daß davon ausgegangen werden kann, daß sich z.B. Diltiazem innerhalb des verträglichen Dosisbereichs von 10 - 100 mg pro orale Einzeldosis zur Bekämpfung von AIDS beim Menschen eignet.

Die Verbindungen I können erfindungsgemäß beim Menschen in flüssiger oder fester Form enteral oder parenteral appliziert werden. Als Injektionsmedium kommen vor allem wäßrige Phasen zur Anwendung, welche die üblichen Zusätze wie Stabilisierungsmittel und Lösungsvermittler enthalten. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Kalziumphosphat, Magnesiumstearat, tierische und pflanzliche Fette, feste hochmolekulare Polymere (wie Polyäthylenglykole); für orale Applikation geeignete Zubereitungen können gewünschtenfalls Geschmacks- und/oder Süßstoffe enthalten.

Neben Diltiazem lassen sich auch folgende Verbindungen erfindungsgemäß einsetzen, die sämtlich aus der DE-PS 18 05 714 bekannt sind:

2-(4-Chlorphenyl)-3-hydroxy-5-($\beta$-dimethylaminoäthyl)-2,3-dihydro-1,5-benzothiazepin-4(5H)-on;

2-(3,4,5-Trimethoxyphenyl)-3-hydroxy-5-($\beta$-dimethylaminoäthyl)-7-chlor-2,3-dihydro-1,5-benzothiazepin-4-(5H)-on-perchlorat;

2-(4-Methylphenyl)-3-hydroxy-5-($\beta$-dimethylaminoäthyl)-7-chlor-2,3-dihydro-1,5-benzothiazepin-4(5H)-on-hydrochlorid;

2-(2,4-Dichlorphenyl)-3-hydroxy-5-($\beta$-dimethylaminoäthyl)-7-chlor-2,3-dihydro-1,5-benzothiazepin-4(5H)-on-hydrochlorid;

2-(4-Methylphenyl)-3-hydroxy-5-($\beta$-dimethylaminoäthyl)-2,3-dihydro-1,5-benzothiazepin-4(5H)-on;

2-(4-Methoxyphenyl)-3-hydroxy-5-($\beta$-dimethylaminoäthyl)-7-chlor-2,3-dihydro-1,5-benzothiazepin-4(5H)-on;

2-(4-Chlorphenyl)-3-hydroxy-5-($\beta$-dimethylaminoäthyl)-7-chlor-2,3-dihydro-1,5-benzothiazepin-4(5H)-on-hydrochlorid;

2-Phenyl-3-hydroxy-5-(β-dimethylaminoäthyl)-2,3-dihydro-1,5-benzothiazepin-4(5H)-on;
2-(3,4-Dimethoxyphenyl)-3-hydroxy-5-(β-dimethylaminoäthyl)-2,3-dihydro-1,5-benzothiazepin-4(5H)-on-hydrochlorid;
2-(3,4,5-Trimethoxyphenyl)-3-hydroxy-5-(β-dimethylaminoäthyl)-2,3-dihydro-1,5-benzothiazepin-4(5H)-on-hydrobromid;
2-(2,4-Dichlorphenyl)-3-hydroxy-5-(β-dimethylaminoäthyl)-2,3-dihydro-1,5-benzothiazepin-4(5H)-on-hydrochlorid;
2-Phenyl-3-hydroxy-5-(β-dimethylaminoäthyl)-7-chlor-2,3-dihydro-1,5-benzothiazepin-4(5H)-on-hydrochlorid;
2-(3,4-Dimethoxyphenyl-3-hydroxy-5-(β-dimethyalminoäthyl)-7-chlor-2,3-dihydro-1,5-benzothiazepin-4(5H)-on-hydrochlorid;
2-(4-Methoxyphenyl)-3-hydroxy-5-(β-dimethylaminoäthyl)-2,3-dihydro-1,5-benzothiazepin-4(5H)-on;
2-Phenyl-3-hydroxy-5-(γ-dimethylamino-n-propyl)-2,3-dihydro-1,5-benzothiazepin-4(5H)-on;
2-(4-Methoxyphenyl)-3-hydroxy-5-(γ-dimethyl-amino-n-propyl)-2,3-dihydro-1,5-benzothiazepin-4(5H)-on-hydrochlorid;
2-(3,4,5-Trimethoxyphenyl)-3-hydroxy-5-(γ-dimethylamino-n-propyl)-2,3-dihydro-1,5-benzothiazepin-4(5H)-on-hydrochlorid;
2-(4-Methylphenyl)-3-hydroxy-5-(γ-dimethyl-amino-n-propyl)-2,3-dihydro-1,5-benzothiazepin-4(5H)-on-hydrochlorid;
2-Phenyl-3-hydroxy-5-(γ-dimethylamino-n-propyl)-7-chlor-2,3-dihydro-1,5-benzothiazepin-4(5H)-on-oxalat;
2-(4-Chlorphenyl)-3-hydroxy-5-(γ-dimethylamino-n-propy)7-chlor-2,3-dihydro-1,5-benzothiazepin-4(5H)-on-hydrochlorid;
2-(4-Methoxyphenyl-3-hydroxy-5-(α-methyl-β-dimethylaminoäthyl)-2,3-dihydro-1,5-benzothiazepin-4(5H)-on-oxalat;
2-(4-Methoxyphenyl)-3-hydroxy-5-(γ-dimethylamino-n-propyl)-7-chlor-2,3-dihydro-1,5-benzothiazepin-4(5H)-on;
2-Phenyl-3-acetoxy-5-(β-dimethylaminoäthyl)-2,3-dihydro-1,5-benzothiazepin-4(5H)-on;
2-(4-Methoxyphenyl)-3-acetoxy-5-(β-dimethylaminoäthyl)-2,3-dihydro-1,5-benzothiazepin-4(5H)-on-hydrochlorid;
2-(3,4,5-Trimethoxyphenyl)-3-acetoxy-5-(β-dimethylaminoäthyl-2,3-dihydro-1,5-benzothiazepin-4(5H)-on-oxalat;
2-(4-Methylphenyl)-3-acetoxy-5-(β-dimethylaminoäthyl)-2,3-dihydro-1,5-benzothiazepin-4(5H)-on-hydrochlorid;
4-Phenyl-3-acetoxy-5-(β-dimethylaminoäthyl)-7-chlor-2,3-dihydro-1,5-benzothiazepin-4(5H)-on-hydrochlorid;
2-(4-Methoxyphenyl)-3-acetoxy-5-(β-dimethylaminoäthyl)-7-chlor-2,3-dihydro-1,5-benzothiazepin-4(5H)-on-hydrochlorid;
2-(4-Chlorphenyl)-3-acetoxy-5-(β-dimethylaminoäthyl)-7-chlor-2,3-dihydro-1,5-benzothiazepin-4(5H)-on-hydrochlorid;
2-Phenyl-3-acetoxy-5-(γ-dimethylamino-n-propyl)-2,3-dihydro-1,5-benzothiazepin-4(5H)-on-hydrochlorid;
2-(4-Methylphenyl)-3-acetoxy-5-(γ-dimethylamino-n-propyl)-2,3-dihydro-1,5-benzothiazepin-4(5H)-on-hydrochlorid;
2-(3,4,5-Trimethoxyphenyl)-3-acetoxy-5-(γ-dimethylamino-n-propyl)-2,3-dihydro-1,5-benzothiazepin-4(5H)-on-hydrochlorid;
2-Phenyl-3-acetoxy-5-(γ-dimethylamino-n-propyl)-7-chlor-2,3-dihydro-1,5-benzothiazepin-4(5H)-on-hydrochlorid;
2-(4-Methoxyphenyl)-4-acetoxy-5-(γ-dimethylamino-n-propyl)-7-chlor-2,3-dihydro-1,5-benzothiazepin-4(5H)-on-hydrochlorid;
2-(4-Chlorphenyl)-3-acetoxy-5-(γ-dimethylamino-n-propyl)-7-chlor-2,3-dihydro-1,5-benzothiazepin-4(5H)-on-hydrochlorid.

## Tabelle I

Einfluß von Diltiazem auf die Aktivität eines lymphozytopenischen (Dinalin) und eines nichtlymphozytopenischen (Cefradin**) Immunsuppressivums

Modell: Hauttransplantat-Überlebenstest, Ratte

Empfänger: Long Evans ♂, Spender: Lewis ♂

Es wird der Tag bestimmt, an dem mindestens 50% der Tiere einer Gruppe beide Transplantate abgestoßen haben

| Prüfsubstanz, Dosierung | Tiere mit 2 abgestoßenen Transplantaten/n (%) | | | | | | | | |
|---|---|---|---|---|---|---|---|---|---|
| | Tag + 9 | 10 | 11 | 12 | 13 | 14 | 15 | 16 | 17 |
| Kontrolle<br>Methocel i.g. | 1/8 (17) | 3/6 (50) | 4/6 (66) | 4/6 (66) | 4/6 (66) | 5/6 (83) | 6/6 (100) | | |
| Dinalin<br>10 mg/kg i.g. Tag -3 - +2<br>5 mg/kg i.g. Tag +3 - +6 | 0/5* (0) | 0/5 (0) | 0/5 (0) | 0/5 (0) | 1/5 (20) | 1/5 (20) | 3/5 (60) | 4/5 (80) | 5/5 (100) |
| Diltiazem<br>200 mg/kg i.g. Tag -3 - +6 | 0/6 (0) | 1/6 (17) | 2/6 (33) | 2/6 (33) | 6/6 (100) | | | | |
| Dinalin<br>10 mg/kg i.g. Tag -3 - +2<br>5 mg/kg i.g. Tag +3 - +6<br>und<br>Diltiazem<br>200 mg/kg i.g. Tag -3 - +6 | 0/5* (0) | 0/5 (0) | 2/5 (40) | 2/5 (40) | 3/5 (60) | 3/5 (60) | 4/5 (80) | 5/5 (100) | |
| Cefradin<br>1000 mg/kg i.g. Tag -3 - +6<br>und<br>Diltiazem<br>200 mg/kg i.g. Tag -3 - +6 | 0/6 (0) | 1/6 (17) | 3/6 (50) | 5/5* (100) | | | | | |

* 1 Tier im Versuchsverlauf gestorben

** Cefradin = 7[D-2-Amino-2-(1,4-cyclohexadien-1-yl)-acetamido]-3-methyl-8-oxo-5-thia-1-azabicyclo[4,2,0]-oct-2-en-2-carbonsäure (Rote Liste 1987, Nr. 10.084)

## Patentansprüche

1. Verwendung von Benzothiazepin-Derivaten der allgemeinen Formel I

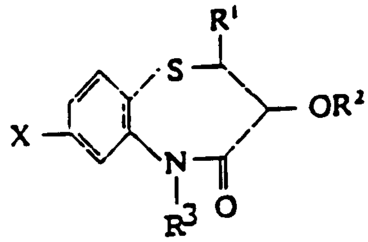

I

in der $R^1$ Wasserstoff oder einen Phenylrest, der gegebenenfalls durch eine Methylgruppe, 1 bis 3 Methoxygruppen oder 1 bis 2 Chloratome substituiert ist, $R^2$ ein Wasserstoffatom oder die Acetylgruppe, X ein Wasserstoffatom oder ein Chloratom und $R^3$ ein Wasserstoffatom oder die Gruppe

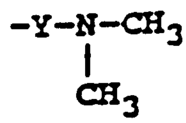

darstellt, wobei Y einen Alkylenrest mit 2 oder 3 Kohlenstoffatomen bedeutet und deren pharmazeutisch verwendbaren Salzen zur Herstellung von Arzneimitteln zur Verhinderung der durch lymphozytotrope Noxen oder Arzneimittel, mit Ausnahme von Zytostatika, hervorgerufene toxische Wirkungen auf Lymphozyten.

2. Verwendung von 2-(4-Methoxyphenyl)-3-acetoxy-5($\beta$-dimethylaminoethyl)-2,3-dihydro-1,5-benzothiazepin-4(5H)-on (Diltiazem) und von dessen pharmazeutisch verwendbaren Salzen nach Anspruch 1.

3. Verwendung von Diltiazem.Hydrochlorid nach Anspruch 1 und 2.

4. Verwendung von Verbindungen gemäß der Ansprüche 1 bis 3 zur Herstellung von Arzneitmitteln zur Behandlung der durch AIDS-Viren hervorgerufene toxischen Wirkungen auf Lymphozyten.

## Claims

1. The use of benzothiazepine derivatives of the general formula:-

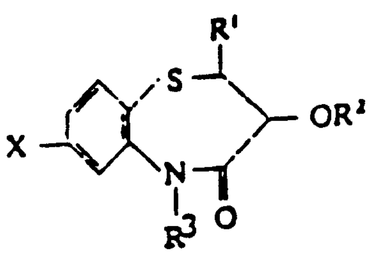

I

wherein $R^1$ is a hydrogen atom or a phenyl radical which is optionally substituted by a methyl group, 1 to 3 methoxy groups or 1 to 2 chlorine atoms, $R^2$ represents a hydrogen atom or the acetyl group, X is a hydrogen atom or a chlorine atom and $R^3$ is a hydrogen atom or the group:-

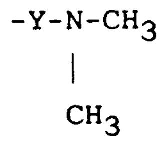

wherein Y is an alkylene radical containing 2 or 3 carbon atoms, and the pharmaceutically acceptable salts thereof for producing medicaments for preventing the toxic effects on lymphocytes caused by lymphocytotopic noxae or medicaments, with the exception of cytostatics.

2. The use of 2-(4-methoxyphenyl)-3-acetoxy-5-(β-dimethylaminoethyl)-2,3-dihydro-1,5-benzothiazepin-4-(5H)-one (diltiazem) and of its pharmaceutically acceptable salts according to claim 1.

3. The use of diltiazem.hydrochloride according to claim 1 and 2.

4. The use of compounds according to claims 1 to 3 for producing medicaments for treating the toxic effects on lymphocytes caused by the AIDS virus.

## Revendications

1. Utilisation de dérivés de la benzothiazépine, répondant à la formule générale (I)

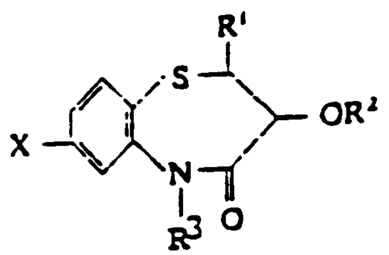

I

dans laquelle $R^1$ représente un atome d'hydrogène ou un radical phényle qui peut être éventuellement substitué par un groupe méthyle, par 1 à 3 groupes méthoxy ou par 1 ou 2 atomes de chlore, $R^2$ représente un atome d'hydrogène ou le groupe acétyle, X représente un atome d'hydrogène ou un atome de chlore et $R^3$ représente un atome d'hydrogène ou le groupe

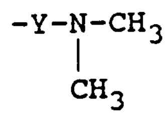

Y représentant un radical alkylène contenant 2 ou 3 atomes de carbone
ainsi que de leurs sels pharmaceutiquement acceptables pour la préparation de médicaments pour la prévention des effets toxiques sur les lymphocytes provoqués par des substances nuisibles lymphocytotropes ou par des médicaments, à l'exception d'agents cytostatiques.

2. Utilisation de la 2-(4-méthoxyphényl)-3-acétoxy-5(β-diméthylaminoéthyl)-2,3-dihydro-1,5-benzothiazépin-4(5H)-one (diltiazem) et de ses sels pharmaceutiquement acceptables selon la revendication 1.

3. Utilisation du chlorhydrate de diltiazem selon les revendications 1 et 2.

4. Utilisation de composés selon les revendications 1 à 3 pour la préparation de médicaments destinés au traitement des effets toxiques sur les lymphocytes, provoqués par les virus du SIDA.

# Figur 1

Haut-Allotransplantat-Überleben (Tage) bei Ratten nach oraler Verabreichung von Dinalin und Diltiazem und deren Kombination, verglichen mit Kontrolle

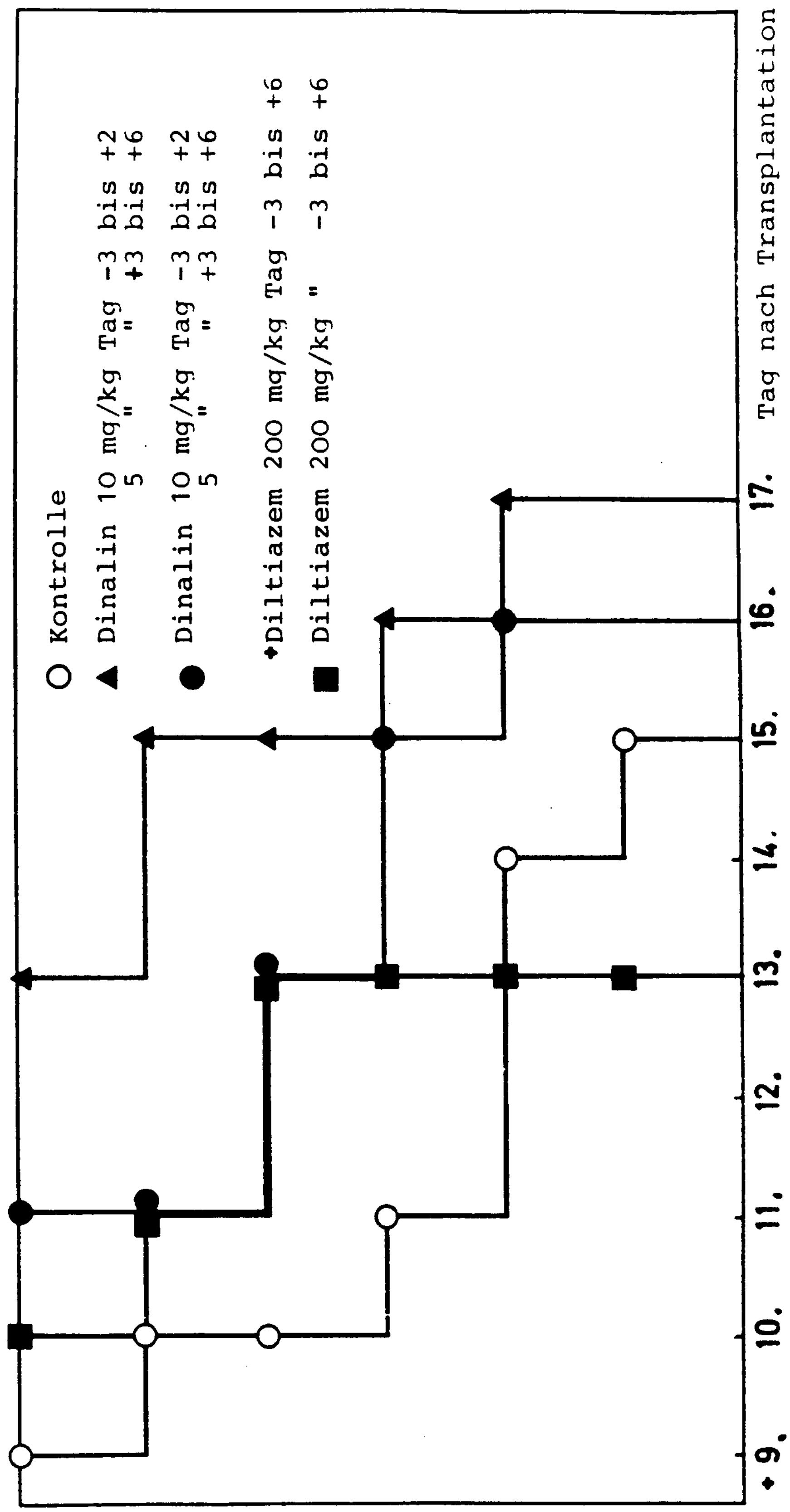

Figur 2

LYMPHOZYTEN IN % GESAMTLEUKOZYTEN

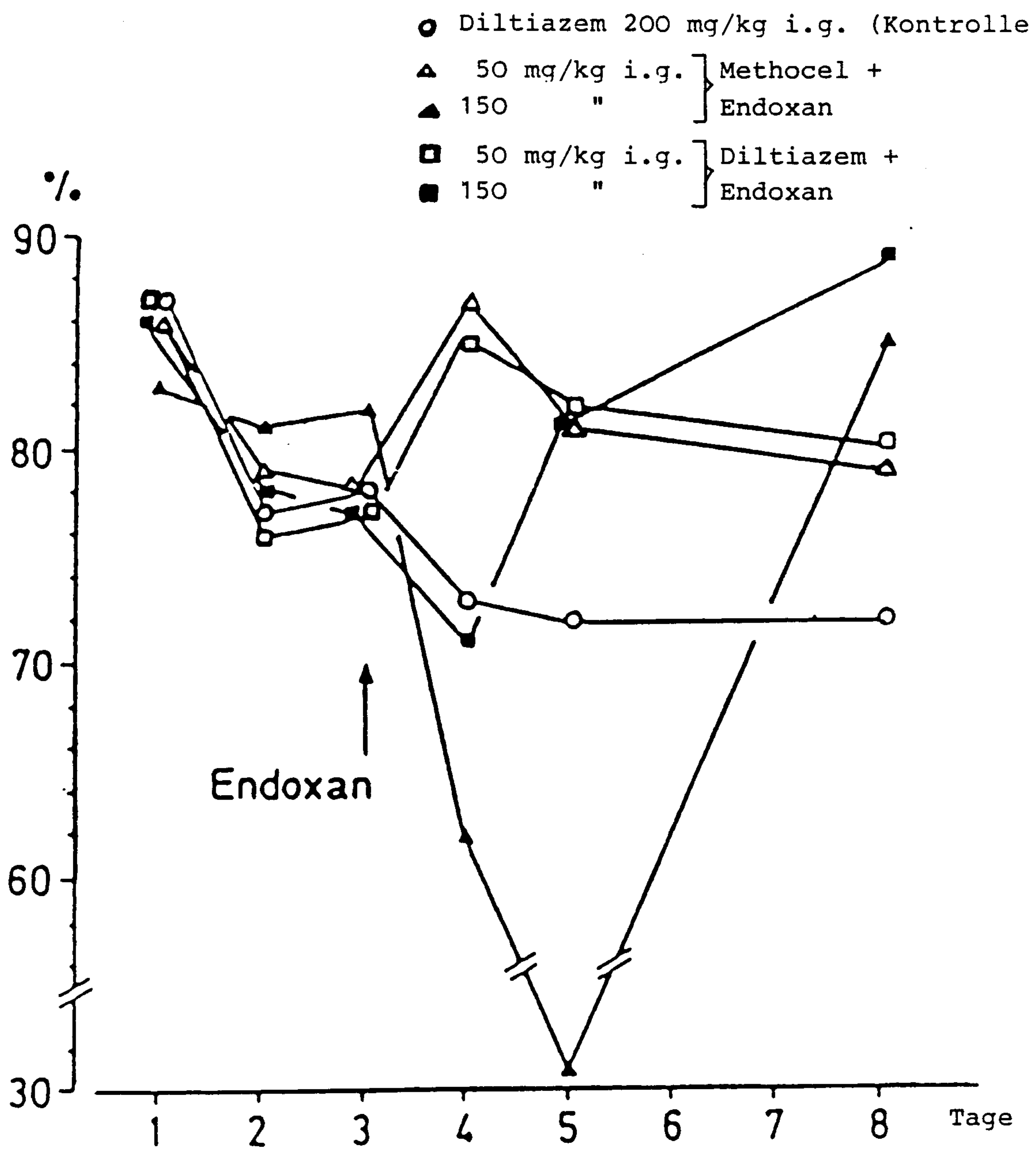

Figur 3

LEUKOZYTENZAHL BEI SD-RATTEN